# EUROPEAN PATENT SPECIFICATION

(11) **EP 1 173 769 B1**
(45) Date of publication and mention of the grant of the patent: **28.02.2007**
(21) Application number: 00929471.1
(22) Date of filing: 02.05.2000
(51) Int. Cl.: G01N 33/68, G01N 33/53

(54) **METHODS OF DIAGNOSING OR TREATING ALZHEIMER'S DISEASE**
METHODEN ZUR DIAGNOSTIZIERUNG ODER ZUR BEHANDLUNG DER ALZHEIMER-KRANKHEIT
PROCEDES PERMETTANT DE DIAGNOSTIQUER OU TRAITER LA MALADIE D'ALZHEIMER

(30) Priority: 03.05.1999 EP 99108722; 09.10.1999 EP 99120211
(43) Date of publication of application: 23.01.2002
(73) Proprietor: Evotec AG, 22525 Hamburg (DE)
(72) Inventor: NITSCH, Roger, 8126 Zumikon (CH); HOCK, Christoph,, 8703 Erlenbach (CH); OTTEN, Uwe,, 4052 Basel (CH)
(74) Representative: Meyers, Hans-Wilhelm
(86) International application number: PCT/EP2000/003913
(87) International publication number: WO 2000/067033

(56) References cited:
- WO-A-91/19982
- WO-A-94/19461
- DICOU E; VERMERSCH P; PENISSON-BESNIER I; DUBAS F; NERRI ERE V: "Anti-NGF autoantibodies and NGF in sera of Alzheimer patients and in normal subjects in relation to age" AUTOIMMUNITY, vol. 26, no. 3, 1997, pages 189-194, XP000852982 cited in the application
- LORIGADOS L; SODERSTROM S; EBENDAL T: "Two-site enzyme immunoassay for beta NGF applied to human patient sera" JOURNAL OF NEUROSCIENCE RESEARCH, vol. 32, no. 3, July 1992 (1992-07), pages 329-339, XP000852933 cited in the application
- MASSARO A R; SORANZO C; BIGON E; BATTISTON S; MORANDI A; CARNEVALE A; CALLEGARO L: "Nerve growth factor (NGF) in cerebrospinal fluid (CSF) from patients with various neurological disorders" ITALIAN JOURNAL OF NEUROLOGICAL SCIENCES, vol. 15, no. 2, March 1994 (1994-03), pages 105-108, XP000852926 cited in the application
- CRUTCHER K A; SCOTT S A; LIANG S; EVERSON W V; WEINGARTNER J: "Detection of NGF-like activity in human brain tissue: increased levels in Alzheimer's disease" JOURNAL OF NEUROSCIENCE, vol. 13, no. 6, June 1993 (1993-06), pages 2540-2550, XP000852929 cited in the application
- HOCK CHRISTOPH; HEESE KLAUS; MUELLER-SPAHN FRANZ; HULETTE CHRISTINE; ROSENBERG CARLYN; OTTEN UWE: "Decreased trkA neurotrophin receptor expression in the parietal cortex of patients with Alzheimer's disease" NEUROSCIENCE LETTERS, vol. 241, 30 January 1998 (1998-01-30), pages 151-154, XP000949445
- SEIGER A, NORDBERG A, VON HOLST H, BACKMAN L, EBENDAL T, ALAFUZOFF I, AMBERLA K, HARTVIG P, HERLITZ A, LILJA A, ET AL: "Intracranial infusion of purified nerve growth factor to an Alzheimer patient: the first attempt of a possible future treatment strategy" BEHAVIOURAL BRAIN RESEARCH, vol. 57, no. 2, 30 November 1993 (1993-11-30), pages 255-261, XP000852665 cited in the application
- B HOFFER, L OLSON: "Treatment strategies for neurodegenerative diseases based on trophic factors and cell transplantation techniques" JOURNAL OF NEURAL TRANSMISSION. SUPPLEMENTUM, vol. 49, 1997, pages 1-10, XP000852673 cited in the application
- LAPCHAK P A: "NERVE GROWTH FACTOR PHARMACOLOGY: APPLICATION TO THE TREATMENT OF CHOLINERGIC NEURODEGENERATION IN ALZHEIMER'S DISEASE" EXPERIMENTAL NEUROLOGY, vol. 124, 1 January 1993 (1993-01-01), pages 16-20, XP002037880 cited in the application
- SOFRONIEW, MICHAEL V.: "Nerve growth factor, ageing and Alzheimer's disease" ALZHEIMER S RESEARCH, vol. 2, no. 1-2, 1996, pages 7-13, XP000852943 cited in the application
- NISHIO T, SUNOHARA N, MIZUTANI K, AKIGUCHI I, FURUKAWA S: "Nerve growth factor levels in cerebrospinal fluid are high in the inflammatory neurological disorders" CLINICA CHIMICA ACTA, vol. 275, no. 1, 6 July 1998 (1998-07-06), pages 93-98, XP000852979 cited in the application
- LAPPALAINEN R; LINDHOLM D; RIIKONEN R: "Low levels of nerve growth factor in cerebrospinal fluid of children with Rett syndrome" JOURNAL OF CHILD NEUROLOGY, vol. 11, no. 4, July 1996 (1996-07), pages 296-300, XP000852954 cited in the application
- WESKAMP G, OTTEN U: "An enzyme-linked immunoassay for nerve growth factor (NGF): a tool for studying regulatory mechanisms involved in NGF production in brain and in peripheral tissues" JOURNAL OF NEUROCHEMISTRY, vol. 48, no. 6, June 1987 (1987-06), pages 1779-1786, XP000852664 cited in the application
- MURASE, KATSUHITO; NABESHIMA, TOSHITAKA; ROBITAILLE, YVES; QUIRION, REMI; OGAWA, MICHIKO; HAYASHI, KYOZO: "NGF level is not decreased in the serum, brain-spinal fluid, hippocampus, or parietal cortex of individuals with Alzheimer's disease" BIOCHEMICAL AND BIOPHYSICAL RESEARCH COMMUNICATIONS, vol. 193, no. 1, 1993, pages 198-203, XP002122252 cited in the application

## Description

Alzheimer's disease (AD), first described by the Bavarian psychiatrist Alois Alzheimer in 1907, is a progressive neuropsychiatric disorder which begins with short term memory loss and proceeds to loss of cognitive functions, disorientation, impairment of judgement and reasoning and, ultimately, dementia. It is the most common form of dementia. The neuropathology is characterized by the formation in brain of amyloid plaques and neurofibrillary tangles. AD has been estimated to afflict 5 to 11 percent of the population over age 65 and as much as 47 percent of the population over age 85. Moreover, as adults, born during the population boom of the 1940's and 1950's, approach the age when AD becomes more prevalent, the control and treatment of AD will become an even more significant health care problem. Familial forms of AD are genetically heterogeneous, but most with early onset are linked to mutations in the presenilin genes *PSEN1* and *PSEN2,* as well as to mutations of the amyloid precursor gene *APP.* The majority of AD patients have no obvious family history and are classified as sporadic AD. For this late onset AD, several putative genetic risk factors have been reported. Among these the ApoE-epsilon 4 (ApoE 4) has been widely confirmed to confer increased risk for AD. Inheritance of ApoE4 and other risk factors are neither necessary nor sufficient to cause AD. In contrast to the APP- and PSEN mutations which increase the production of Aβ, the principal component of senile plaques in AD brain, the ApoE variant most likely influences Aβ accumulation by modulating clearance and degradation of the peptide.

In the search for biochemical changes in patients with neuropsychiatric and neurodegenerative disorders analysis of cerebrospinal fluid (CSF) may be a useful method, since the CSF is continuous with the extracellular fluid of the brain. Therefore, a plurality of studies aiming at the analysis of the central nervous system (CNS) specific proteins in CSF were performed in order to find biochemical markers for neuronal and synaptic function and pathology in degenerative brain disorders.

Nerve growth factor (NGF) is one of the neurotrophic agents that promote differentiation or support the survival and functioning of some populations of neurons, influencing their effects not only on the peripheral sensory and sympathetic neurons but also on the central neurons. The pathophysiological role of NGF in the human nervous system, especially in relation to neuropsychiatric disorders, has not been fully understood yet. It is known that patients with acute multiple sclerosis (MS), traumatic brain injury or hypertensive cerebral hemorrhage show higher NGF levels in the CSF and NGF has trophic roles in regenerating axons in the CNS.

To determine the pathophysiological roles of NGF in the human CNS with special reference to neuropsychiatric disorders, levels of NGF in CSF from patients with the following neurodegenerative disorders have been examined by Nisho et al. (Clinica Chimica Acta 275, 93 - 98, 1998) using a highly sensitive two-site enzyme immunoassay:
(i) Parkinson's disease
(ii) Progressive supranuclear palsy
(iii) Sporadic olivo-ponto-cerebellar atrophy
(iv) Spinocerebellar ataxia 3 / Machado-Joseph disease
(v) Dentato-rubro-pallido-luysian atrophy

However, Nisho et al. did not examine any patients suffering from Alzheimer's disease.

Lappalainen et al. (Journal of Child Neurology 11 (4), 296 - 300, 1996) report about low levels of NGF in cerebrospinal fluid of children with Rett Syndrome.

Dicou et al. (Autoimmunity 26 (3), 189 - 194, 1997) report that no changes in anti-NGF autoantibody titers or in NGF frequency are detected in sera of AD patients, suggesting that they are not involved in the neuroimmunological mechanisms underlying AD.

Lorigados et al. (Journal of Neuroscience Research 32 (3), 329 - 339, 1992) applied a two-site enzyme immunoassay to examine NGF levels in normal human serum and serum from Alzheimer patients.

Massaro et al. (Italian Journal of Neurological Science 15 (2), 105 - 108, 1994) studied NGF in cerebrospinal fluid from patients with various neurological disorders including AD. Their study does not support the possibility that NGF is involved in the neuroimmunological mechanisms which can be expected to be linked in the inflammatory or degenerative diseases of the central and peripheral nervous system chosen in their study.

Crutcher et al. (The Journal of Neuroscience 13 (6), 2540 - 2550, 1993) used a two-site ELISA and a bioassay to detect NGF-like activity in human brain tissue. NGF-like activity was significantly elevated in the frontal and occipital cortex from patients with AD. Their results demonstrate the feasibility of detecting NGF-like activity in both fresh and postmortem human brain tissue.

The international patent application PCT/EP 91/01100 discloses a method for the qualitative and quantitative determination of a polypeptide or protein analyte present in a biological fluid or a solution. This method is exemplified by the determination of NGF.

Seiger et al. (Behavioural Brain Research 57 (2), 255 - 261, 1993) report on the clinical outcome of a first case of intracranial infusion of NGF to an AD patient. This therapeutic attempt is based on animal research showing that NGF stimulates central cholinergic neurons of the type known to be lost during the development of AD.

Hoffer et al. (Journal of Neural Transmission, Suppl. 49, 1 - 10, 1997) disclose treatment strategies based on transfer of genes, molecules, or cells to the central nervous system. Before degeneration has occurred, it may be possible to rescue "stressed" neurons, and stimulate terminal outgrowth using treatment with neurotrophic factors. Such approaches, with an emphasis on the NGF family of neurotrophins and their receptors, are reviewed.

Lapchak (Experimental Neurology 124, 16 - 20, 1993) provides in his review an overview of the importance of NGF as a neurotrophic factor for adult cholinergic neurons of the septohippocampal pathway. Information concerning the possible therapeutic use of NGF or small molecules that increase the expression of NGF to treat the cholinergic neurodegeneration that occurs in AD are provided.

Sofroniew (Alzheimer's Research 2 (1-2), 7 - 13, 1996) discloses data from pilot studies of NGF infusion into the CSF of patients with AD, peripheral administration of NGF, which suggest that achieving a method for site specific delivery of NGF in the CNS may be an important consideration in developing a treatment strategy.

Murase et al. (Biochemical and Biophysical Research Communications 193 (1), 198 - 203, 1993) disclose that NGF level is not decreased in the serum, brain-spinal fluid, hippocampus, or parietal cortex of individuals with AD.

As AD is a growing social and medical problem, there is a strong need for *ante mortem* methods of diagnosing or prognosing said disease in subjects as well as for methods of treatment.

In one aspect, the invention features a method for diagnosing or prognosing Alzheimer's disease in a subject, or determining whether a subject is at increased risk of developing Alzheimer's disease, comprising:
determining a level of nerve growth factor in a sample taken from cerebrospinal fluid of said subject;
and comparing said level to a reference value representing a known disease or health status,
   wherein a level of nerve growth factor 4 pg/ml in said cerebrospinal fluid from said subject indicates a diagnosis, or prognosis, or increased risk of Alzheimer's disease in said subject.

In a further aspect, the invention features a method of monitoring progression of Alzheimer's disease in a subject, comprising:
determining a level of nerve growth factor in a sample taken from cerebrospinal fluid of said subject;
and comparing said level to a reference value representing a known disease or health status,
wherein a level of nerve growth factor 4 pg/ml in said cerebrospinal fluid from said subject indicates a diagnosis, or prognosis, or increased risk of Alzheimer's disease in said subject.

In still a further aspect, the invention features a method of evaluating a treatment for Alzheimer's disease, comprising:
determining a level of nerve growth factor in a sample taken from cerebrospinal fluid of a subject;
and comparing said level to a reference value representing a known disease or health status,
wherein a level of nerve growth factor ≥ 4 pg/ml in said cerebrospinal fluid from said subject indicates a diagnosis, or prognosis, or increased risk of Alzheimer's disease in said subject.

An increase of a level of nerve growth factor in cerebrospinal fluid from a subject relative to said reference value representing a known health status indicates a diagnosis, or prognosis, or increased risk of said Alzheimer's disease in said subject. In particular, a level of nerve growth factor ≥ 4 pg/ml in said cerebrospinal fluid indicates a diagnosis, or prognosis, or increased risk of Alzheimer's disease in said subject. Specifically, a level of NGF in the range from 4 pg/ml to 25 pg/ml, in particular in the range from 4 pg/ml to 14 pg/ml, in said cerebrospinal fluid indicates a diagnosis, or prognosis, or increased risk of AD in said subject. Said subject is preferably a human. NGF can e.g. be detected using an immunoassay, a bioassay or a binding assay (see e.g. Crutcher et al., The Journal of Neuroscience 13 (6), 2540 - 2550, 1993).

It is particularly preferred to further compare a level of nerve growth factor with a level of NGF in a series of samples taken from said subject over a period of time. Said subject might have received a treatment prior to one or more of said sample gatherings. Said level is preferably determined before and after said treatment.

In a further preferred embodiment, additionally a level of a further neurotrophin, e.g. neurotrophin 3 (NT-3), is determined with the goal of diagnosing, prognosing, evaluating the risk of developing, evaluating a treatment of, or monitoring the progression of Alzheimer's disease. In particular, a level of neurotrophin 3 ≥ 15 pg/ml indicates a diagnosis, or prognosis, or increased risk of Alzheimer's disease in said subject.

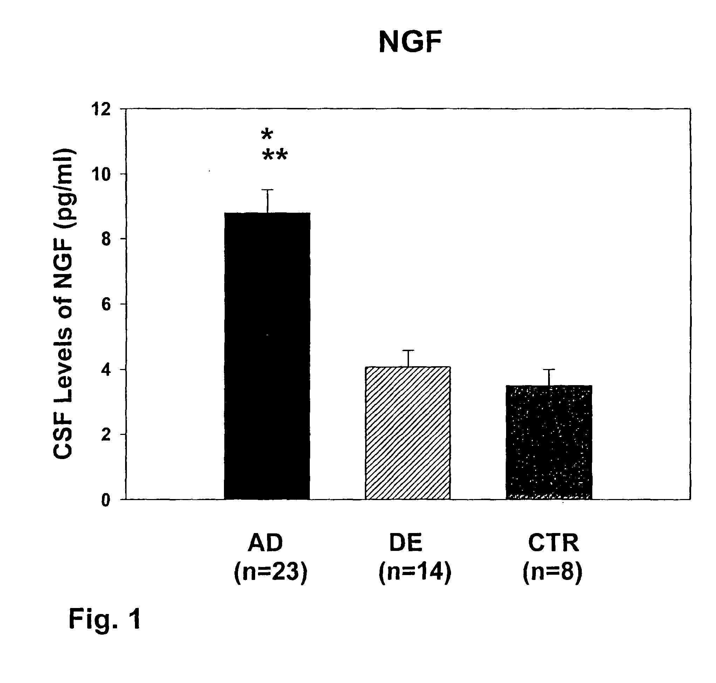

Figure 1 relates to example 1 and depicts that CSF levels of NGF are significantly elevated in the AD group, as compared to both the group consisting of patients with major depression (DE) as well as to the control group (CTR). Levels (pg/ml) are given in mean ± SEM. Asterisk (*, **) indicate significance (p< 0.05), Mann-Whitney U Test. * AD versus DE, p < 0.001; ** AD versus CTR, p < 0.001. NGF concentrations in CSF of the AD group amounted to 8.79 +/- 0.72 pg/ml (mean +/- SEM, range: 3.29 to 14.95, n = 23), compared to 4.07 +/- 0.50 pg/ml in the DE group (range: 2.42 to 9.54, n = 14), and 3.49 +/- 0.51 pg/ml in the CTR group (range: 0.00 to 4.64, n = 8), respectively. The alterations in patients suffering from AD may reflect disturbances in the trophic support of specific neuronal populations, such as the basal forebrain cholinergic system. There was no apparent correlation of CSF levels of NGF with ApoE genotype (or phenotype, respectively), age, duration of AD, MMS, NOSGER or MADRS scores.

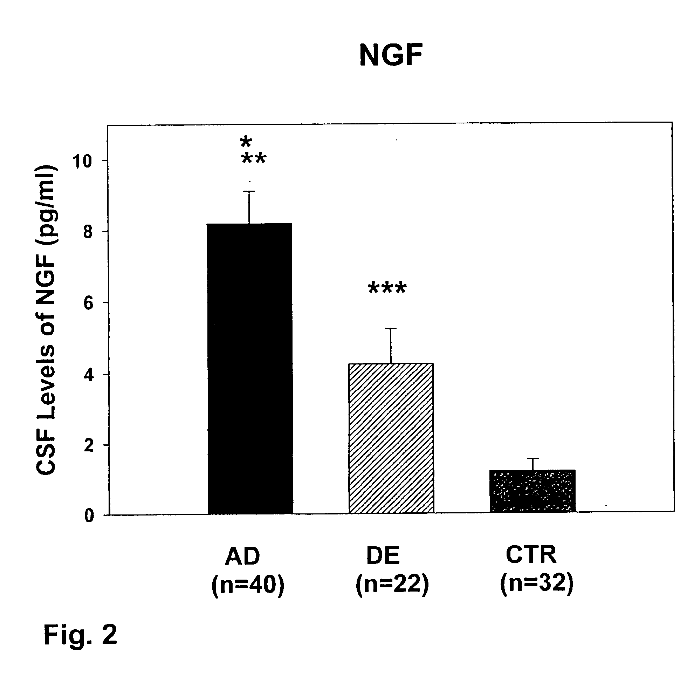

Figure 2 relates to example 2 and depicts nerve growth factor levels in the cerebrospinal fluid of patients with Alzheimer's disease (AD), major depression in the elderly (DE) and non-demented control subjects. Levels (pg/ml) are given in mean ± SEM. Asterix (*, **, ***) indicate significance (p < 0.05), Mann-Whitney U Test. * AD versus DE, p = 0.002; ** AD versus CTR, p = 0.000, *** DE versus CTR, p = 0.000. CSF levels of NGF were significantly elevated in the AD group, as compared to both the DE and the CTR group. CSF levels of NGF were also significantly elevated in the DE group, as compared to the CTR group. NGF concentrations in CSF of the AD group amounted to 8.19 +/- 0.91 pg/ml (mean +/- SEM, range: 0.00 to 23.00, n = 40), compared to 4.26 +/- 0.97 pg/ml in the DE group (range: 0.00 to 23.00, n = 22), and 1.18 +/- 0.35 pg/ml in the CTR group (range: 0.00 to 7.20, n = 32), respectively.

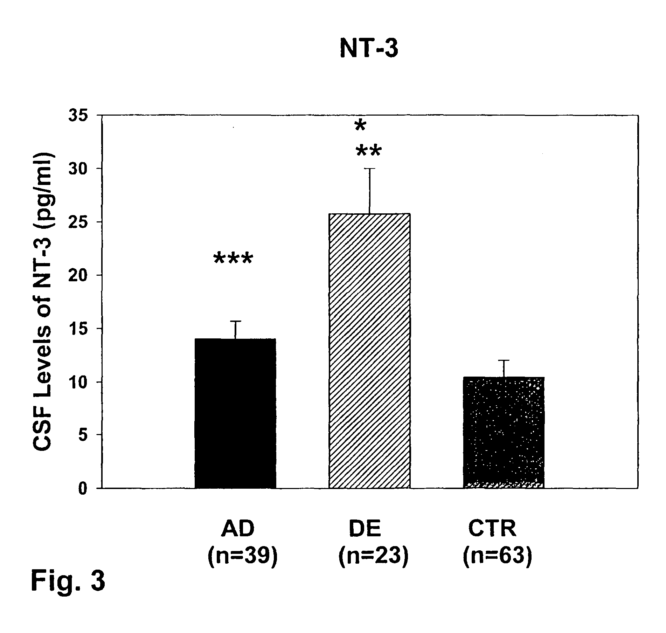

Figure 3 relates to example 3 and depicts neurotrophin 3 (NT-3) levels in the cerebrospinal fluid of patients with Alzheimer's disease (AD), major depression in the elderly (DE) and non-demented control subjects (CTR). CSF levels of NT-3 were determined to define a cut-off value to be used in the combined tests shown in table 1. Levels (pg/ml) are given in mean ± SEM. Asterix (*, **, ***) indicate significance (p < 0.05), Mann-Whitney U Test. * DE versus AD, p = 0.005; ** DE versus CTR, p = 0.000; *** AD versus CTR, p = 0.010. CSF levels of NT-3 were significantly elevated in the DE group, as compared to both the AD and the CTR group. CSF levels of NT-3 were slightly, but significantly, elevated in the AD group, as compared to the CTR group. NT-3 concentrations in CSF of the DE group were 25.8 +/- 4.3 pg/ml (mean +/-SEM, range: 0.0 to 87.0, n =23), compared to 14.0 +/- 1.6 pg/ml in the AD group (range: 0.0 to 41.0, n = 39), and 10.5 +/- 1.6 pg/ml in the CTR group (range: 0.0 to 67.0, n = 63), respectively.

Table 1 relates to examples 2 and 3. This table shows the diagnostic accuracy of spinal fluid measurements of NGF and NT-3 in Alzheimer's Disease and Major Depression in the Elderly. In NGF measurements, sensitivity, specificity, positive predictive value (PPV) and negative predictive value (NPV) were calculated using a cut-off value of ≥ 4.0 pg/ml NGF (total: n = 94, AD: n = 40, DE: n = 22, CTR: n = 32). The combined NGF/NT-3 test showed a considerable specificity for the diagnosis of AD (90.1 %), using cut-off values of ≥ 4 pg/ml NGF, and < 15 pg/ml NT-3, respectively (total: n = 57, AD: n = 24, DE: n = 18, CTR: n = 15). Testing either NGF levels or NGF and NT-3 levels with suitable cut-off criteria constitutes candidate tools for specific biochemical diagnosis of AD. Using the opposite cut-off criteria, the combination test significantly separated AD patients from elderly DE patients with a specificity of 89.7 %. Therefore, another potential use of this test is the biochemical differentiation between these two frequent disorders in the elderly.

Table 2 depicts the clinical characteristics and test scores of patients with Alzheimer's disease (AD), major depression (DE) and non-demented control subjects (CTR). Nerve growth factor (NGF), neurotrophin 3 (NT-3), MMS (Mini Mental State), MADRS (Montgomery Asberg Depression Rating Scale), ApoE (apolipoprotein E), n.d. (not determined).

### EXAMPLE 1

In order to achieve a differential diagnosis, the study included not only patients with AD, but also such with major depression (DE). Diagnosis of probable AD was made according to criteria of the National Institute of Neuropsychiatric and Communicative Disorders and Stroke-Alzheimer's disease and Related Disorders Association (NINCDS-ADRDA; McKhann et al., Neurology 34, 939 - 944, 1984). Patients with major depression were diagnosed according to the ICD10 (F32.0x/1x, F33.0x/1x) and DSM-IIIR (296.20-22, 296.30-32) criteria. All patients were referred to the research ward from general practitioners, neurologists and psychiatrists for diagnostic purposes and screening for clinical trials. None of the patients was institutionalized. The group of healthy control subjects (CTR) consisted of patients that underwent lumbar puncture for orthopedic or neurologic diagnostic purposes and were shown to have normal CSF cell counts, total protein levels, and absence of signs of blood barrier dysfunction or cerebral IgG synthesis, as well as absence of any cerebral disorders.

AD, DE and CTR patients were carefully examined and received a thorough clinical work-up. Psychometric testing including the Mini Mental State (MMS; Folstein et al., J. Psychiatry Res. 12, 189 - 198, 1975), as a global screening instrument for dementia, and the Nurses' Observation Scale for Geriatric Patients (NOSGER; Spiegel et al., J. Am. Geriatr. Soc. 39(4), 339 - 347, 1991) as a functional measure of dementia severity. The patients with DE showed no cognitive disturbances in the clinical examinations and the Mini Mental State scores were within the normal range. Severity of depression was rated by using the Montgomery Asberg Depression Rating Scale (MADRS) (Montgomery et al., Br. J. Psychiatry, 134: 382 - 389, 1979). Apolipoprotein (ApoE) genotyping, or, if DNA was not available, ApoE phenotyping was included in the laboratory screening in the AD patients.

CSF was obtained for diagnostic purposes in the AD and DE patients in which no lumbar puncture had been previously done during the routine diagnostic work-up. Different CSF volumes were available for the analysis of the neurotrophin proteins. This fact explains the different sample sizes for the individual measurements. All available CSF samples were used for the analyses.

The AD group was as follows: n = 23, 12 men, 11 women, mean age 63.9 +/-13.2 SD years, range 39 - 86 years, MMS score: mean 18.6 +/- 5.6 SD.

The DE group was as follows: n = 14, 5 men, 9 women, mean age 68.2 +/-13.6 SD years, range 47 - 86 years, MMS score: mean 28.1 +/- 0.9 SD.

The CTR group was as follows: n = 8, 5 men, 3 women, mean age 60.1 +/-18.1 SD years, range 31 - 81 years.

AD and CTR patients were free of psychotropic medication. Patients with major depression were treated with various antidepressant drugs including serotonin reuptake inhibitors, reversible monoaminooxidase A inhibitors and tricyclics. Informed consent was taken from each patient and their caregivers before the investigation. The study was approved by the local ethics committee. All procedures were in accordance with the Helsinki Declaration of 1975, as revised in 1983. Within one week of dementia testing, CSF was obtained by lumbar puncture. To control for possible influences of a ventriculo-lumbar gradient, lumbar punctures were done between 7.30 and 8 a. m. before breakfast while patients were still lying flat. CSF samples were frozen on dry ice immediately upon withdrawal at the bedside in 0.5 ml aliquots and stored at - 85 °C until biochemical analysis.

CSF levels of NGF were measured by an ELISA as described recently (Weskamp et al., J. Neurochem. 48, 1779 - 1786, 1987). Black 96-well microplates (Nunc) were coated with monoclonal anti-β (2.5 S, 7S) NGF antibodies (Ab) (clone 27/21, Boehringer Mannheim) diluted in carbonate buffer pH 9.2 over night at 4 °C. 120 µl of CSF and standard solutions were added and incubated for 20 hours at 4 °C. Plates were washed and incubated with anti-β (2.5 S, 7S) NGF-β-galactosidase conjugate for 2 ½ hours at room temperature (RT). Following an additional washing step, the fluorogenic substrate 4-methylumbelliferyl-β-D-galactopyranoside was added and plates were incubated at 4 °C over night. The reaction was stopped after 1 h at RT and the flurorescent product was measured in the microtiter wells using a fluorometer (Labsystems Fluoroskan Ascent FL) (excitation wavelength: 355 nm; emission wavelength: 460 nm). The detection limit was 1.5 pg/ml; the cross-reactivity with other neurotrophins at 10 ng/ml was < 2 %.

ApoE genotyping was performed using INNO-LiPA ApoE, Innogenetics, Belgium. ApoE phenotyping was performed according to McDowell et al. (Clin. Chem. 35(10), 2070 - 2073, 1989). The use of the ApoE phenotype synonymous with the ApoE genotype in the statistical analyses seemed to be appropriate, since ApoE genotyping compared with protein phenotyping showed conflicting results in less than 2 % (Hansen et al., Clin. Chim. Acta, 224(2), 131 - 137, 1994).

Statistical analyses of data were performed using the Mann-Whitney U test for group comparisons. Correlation analyses were performed by multiple regression using CSF levels of neurotrophins as well as ApoE genotype (or phenotype, respectively), age, duration of the disease in AD, MMS, NOSGER and MADRS scores. Regression analysis was complemented with analysis of variance (ANOVA) by using SPSS for Windows (version 8.0). Statistical significance was assumed at p < 0.05. Bonferroni correction for multiple testing was applied.

### EXAMPLE 2

The study described in example 1 has been extended to a wider panel of patients as described below in this example 2.

Diagnosis, clinical examination and treatment of patients as well as lumbar puncture were performed as described in example 1.

For NGF measurements, 94 spinal fluid samples were examined. The AD group (n = 40) consisted of 18 men and 22 women, mean age 68.8 +/- 12.4 SD years, range 39 - 88 yr, MMS score: mean 19.3 +/- 4.6 SD. The DE group (n = 22) consisted of 8 men and 12 women, mean age 69.8 +/- 12.6 SD years, range 47 - 86 yr, MMS score: mean 27.5 +/- 2.1 SD. CTR group: n = 32, 18 men, 14 women, mean age 64.0 +/- 14.9 SD years, range 29 - 96 yr.

CSF levels of NGF were measured by an ELISA as described by Weskamp et al. (J. Neurochem. 48: 1779 - 1786, 1987). Black 96-well microplates (Nunc) were coated with monoclonal anti-β (2.5 S, 7S) NGF antibodies (Ab) (clone 27/21, Boehringer Mannheim) diluted in carbonate buffer pH 9.2 overnight at 4 °C. 120 µl of CSF and standard solutions were added and incubated for 20 hours at 4 °C. Plates were washed and incubated with anti-β (2.5 S, 7S) NGF-β-galactosidase conjugate for 2 ½ hours at room temperature (RT). Following an additional washing step, the fluorogenic substrate 4-methylumbelliferyl-β-D- galactopyranoside was added and plates were incubated at 4 °C overnight. The reaction was stopped after 1h at RT, and the fluorescent product was measured in the microtiter wells by using a fluorometer (Labsystems Fluoroskan Ascent FL) at 355 nm excitation and 460 nm emission wavelength. The detection limit was 0.5 pg/ml; the cross-reactivity with other neurotrophins at 10 ng/ml was < 2 % and the assay was linear over a range of 0.5 to 500 pg/ml.

Statistical analyses of data were performed using the Mann-Whitney U test for group comparisons. Regression analysis was complemented with analysis of variance (ANOVA) by using SPSS for Windows (version 8.0). Statistical significance was assumed at p < 0.05. Bonferroni correction for multiple testing was applied.

To estimate the diagnostic accuracy of the test, a) sensitivities and b) specificities, defined as follows, were calculated: a) true positives / (true positives and false negatives), and b) true negatives / (true negatives and false positives). To estimate the probability of disease, predictive values of the tests were calculated. The positive predictive value (PPV) was defined as true positives / (true positives + false positives). The negative predictive value (NPV) was defined as true negatives / (true negatives + false negatives).

### EXAMPLE 3

The purpose of this study was to check whether combined measurements of the CSF levels of NGF and neurotrophin-3 (NT-3) - which also belongs to the group of neurotrophins - improves the diagnostic accuracy of the NGF test described in example 2.

In a first step, NT-3 levels in CSF were determined to define a cut-off value to be used in the combined tests. Diagnosis, clinical examination and treatment of patients, lumbar puncture and statistical analyses were performed as described in example 2. For NT-3 measurements, 125 spinal fluid samples were examined. AD group: n = 39, 20 men, 19 women, mean age 67.2 +/-11.5 SD years, range 39 - 86 yr, MMS score: mean 19.1 +/- 5.3 SD. DE group: n = 23, 8 men, 15 women, mean age 70.5 +/- 11.9 SD years, range 47 - 86 yr, MMS score: mean 27.2 +/- 2.5 SD. CTR group: n = 63, 35 men, 28 women, mean age 56.0 +/- 15.0 SD years, range 28 - 84 yr. NT-3 was determined by using commercially available ELISA systems (Promega, Madison, WI) according to the manufacturer's protocol. 120 µl of undiluted CSF in carbonate buffer (pH 9.7) were added to 96 well immunoplates (Nunc) at 4 °C overnight. Anti-Human-NT-3 polyclonal antibodies (pAb) were used as capture Ab. Anti-NT-3 mAb were used as reporter Ab. After incubation with a species-specific Ab (anti-rat IgG) conjugated to horseradish peroxidase (HRP) as a tertiary reactant, and washing, the solution was incubated with the chromogenic substrate TMB (3, 5, 3', 5'-tetramethylbenzidine). Absorbance was measured at 450 nm by using a microplate reader (Dynatech MR 700). NT-3 ELISA: linear range 4.7 - 300 pg/ml; cross-reaction with other neurotrophins at 10 ng/ml < 3%; detection limit 6.0 pg/ml.

57 CSF samples were available for combined NGF/NT-3 measurements. AD group: n = 24, 13 men, 11 women, mean age 64.9 +/- 12.5 SD years, range 47 - 82 yr, MMS score: mean 18.6 +/- 5.4 SD. DE group: n = 18, 7 men, 11 women, mean age 69.5 +/- 12.7 SD years, range 47 - 84 yr, MMS score: mean 27.7 +/- 2.1 SD. CTR group: n = 15, 10 men, 5 women, mean age 59.0 +/- 15.9 SD years, range 29 - 80 yr.

**Table 1**

| **Spinal Fluid Measurements of NGF and NT-3: Diagnostic Accuracy in Alzheimer's Disease (AD) and Major Depression in the Elderly (DE)** | | |
|---|---|---|
| | **NGF test** (NGF>4 pg/ml) | **NT-3 test** (NT-3>15 pg/ml) |
| | **Diagnosis of AD** | **Diagnosis of DE** |
| **Sensitivity** | 71,9% | 73,9% |
| **Specificity** | 79,2% | 86,1 % |
| **Positive Predictive Value (PPV)** | 67,6% | 50,0% |
| **Negative Predictive Value (NPV)** | 82,4% | 91,7% |

| | **Combined NGF/NT-3 test** (NGF>4 pg/ml, NT-3<15 pg/ml) | **Combined NGFINT-3 test** (NT-3>15 pg/ml, NGF<4 pg/ml) |
|---|---|---|
| | **Diagnosis of AD** | **Diagnosis of DE** |
| **Sensitivity** | 62.5% | 55.5% |
| **Specificity** | 90.1% | 89.7% |
| **Positive Predictive Value (PPV)** | 83.3% | 71.4% |
| **Negative Predictive Value (NPV)** | 76.9% | 81.4% |

**Table 2**

| **Clinical Characteristics** | | | | |
|---|---|---|---|---|
| | | ***AD*** | ***DE*** | ***CTR*** |
| NGF measurements | n | 40 (18 m, 22 f) | 21 (8 m, 13 f) | 32 (18 m, 14 f) |
| | Age (mean±SD) yrs | 68.8±12.4 | 69.3±12.6 | 64.0±14.9 |
| | Range (yrs) | 39-88 | 47-86 | 29-96 |
| | MMS score (mean±SD) | 19.3±4.6 | 27.6±2.1 | n.d. |
| | MADRS score | n.d. | 18.6±9.7 | n.d. |
| | ApoE (%) | 2/3 (14), 3/3 (36) | 2/3 (23), 3/3 (34) | n.d. |
| | | 3/4 (43), 4/4 (7) | 3/4 (31), 4/4 (12) | |
| NT-3 measurements | n | 39 (20 m, 19 f) | n.d. | 63 (35 m, 28 f) |
| | Age (mean±SD) yrs | 67.2±11.5 | | 56.0±15.0 |
| | Range (yrs) | 39-86 | | 28-84 |
| | MMS score (mean±SD) | 19.1±5.3 | | n.d. |
| | MADRS score | n.d. | | n.d. |
| | ApoE (%) | 2/3 (14), 3/3 (34) | | n.d. |
| | | 3/4 (45), 4/4 (7) | | |

## Claims

1. A method for diagnosing or prognosing Alzheimer's disease in a subject, or determining whether a subject is at increased risk of developing Alzheimer's disease, comprising:
determining a level of nerve growth factor in a sample taken from cerebrospinal fluid of said subject;
and comparing said level to a reference value representing a known disease or health status,
wherein a level of nerve growth factor ≥ 4 pg/ml in said cerebrospinal fluid from said subject indicates a diagnosis, or prognosis, or increased risk of said Alzheimer's disease in said subject.

2. A method of monitoring progression of Alzheimer's disease in a subject, comprising:
determining a level of nerve growth factor in a sample taken from cerebrospinal fluid of said subject;
and comparing said level to a reference value representing a known disease or health status,
wherein a level of nerve growth factor ≥ 4 pg/ml in said cerebrospinal fluid from said subject indicates a diagnosis, or prognosis, or increased risk of said Alzheimer's disease in said subject.

3. Use of the method according to claims 1 or 2 for evaluating a treatment for Alzheimer's disease.

4. The method according to claim 1 to 3, wherein a level of nerve growth factor in the range from 4 pg/ml to 25 pg/ml, in particular in the range from 4 pg/ml to 14 pg/ml, in said cerebrospinal fluid indicates a diagnosis, or prognosis, or increased risk of Alzheimer's disease in said subject.

5. The method according to any of claims 1 to 4, wherein said subject is a human.

6. The method according to any of claims 1 to 5, wherein nerve growth factor is detected using an immunoassay, bioassay and/or binding assay.

7. The method according to any of claims 1 to 6, further comprising comparing a level of nerve growth factor in said sample with a level in a series of samples taken from said subject over a period of time.

8. The method according to any of claims 1 to 7, wherein said subject receives a treatment prior to one or more of said sample gatherings.

9. The method according to any of claims 1 to 8, wherein said level in said samples is determined before and after said treatment of said subject.

10. The method according to any of claims 1 to 9, further comprising:
determining a level of a further neurotrophin in a sample taken from cerebrospinal fluid of said subject;
and comparing said level to a reference value representing a known disease or health status;
wherein a varied level of said further neurotrophin in said cerebrospinal fluid from said subject relative to said reference value representing a known health status indicates a diagnosis, or prognosis, or increased risk of Alzheimer's disease in said subject.

11. The method according to claim 10 wherein said neurotrophin is neurotrophin-3.

12. The method according to claim 11 wherein a level of neurotrophin-3 ≥ 15 pg/ml in said cerebrospinal fluid indicates a diagnosis, or prognosis, or increased risk of Alzheimer's disease in said subject.

## Patentansprüche

1. Verfahren zum Diagnostizieren oder Prognostizieren der Alzheimer-Krankheit bei einem Patienten oder zum Bestimmen, ob ein Patient ein erhöhtes Risiko hat, Alzheimer-Krankheit zu entwickeln, umfassend:
Bestimmen einer Konzentration von Nervenwachstumsfaktor in einer Probe, die aus Liquor des Patienten erhalten wurde; und
Vergleichen der Konzentration mit einem Referenzwert, der einen bekannten Krankheits- oder Gesundheitszustand darstellt;
wobei eine Konzentration des Nervenwachstumsfaktors von ≥ 4 pg/ml in dem Liquor von dem Patienten eine Diagnose oder Prognose oder ein erhöhtes Risiko für Alzheimer-Krankheit bei dem Patienten anzeigt.

2. Verfahren zur Überwachung des Fortschritts von Alzheimer-Krankheit bei einem Patienten, umfassend:
Bestimmen einer Konzentration von Nervenwachstumsfaktor in einer Probe, die aus Liquor des Patienten erhalten wurde; und
Vergleichen der Konzentration mit einem Referenzwert, der einen bekannten Krankheits- oder Gesundheitszustand darstellt;
wobei eine Konzentration des Nervenwachstumsfaktors von ≥ 4 pg/ml in dem Liquor von dem Patienten eine Diagnose oder Prognose oder ein erhöhtes Risiko für Alzheimer-Krankheit bei dem Patienten anzeigt.

3. Verwendung des Verfahrens gemäß den Ansprüchen 1 oder 2 zur Bewertung einer Behandlung von Alzheimer-Krankheit.

4. Verfahren gemäß Anspruch 1 bis 3, wobei eine Konzentration des Nervenwachstumsfaktors im Bereich von 4 pg/ml bis 25 pg/ml, insbesondere im Bereich von 4 pg/ml bis 14 pg/ml, in dem Liquor eine Diagnose oder Prognose oder ein erhöhtes Risiko für Alzheimer-Krankheit bei dem Patienten anzeigt.

5. Verfahren gemäß Anspruch 1 bis 4, wobei der Patient ein Mensch ist.

6. Verfahren gemäß einem der Ansprüche 1 bis 5, wobei der Nervenwachstumsfaktor mit Hilfe eines Immunoassays, Bioassays und/oder Bindungsassays nachgewiesen wird.

7. Verfahren gemäß einem der Ansprüche 1 bis 6, das weiterhin das Vergleichen der Konzentration des Nervenwachstumsfaktors in der Probe mit einer Konzentration in einer Reihe von Proben, die im Laufe der Zeit von dem Patienten genommen wurden, umfasst.

8. Verfahren gemäß einem der Ansprüche 1 bis 7, wobei der Patient vor einer oder mehreren der Probenahmen eine Behandlung erhält.

9. Verfahren gemäß einem der Ansprüche 1 bis 8, wobei die Konzentration in den Proben vor und nach der Behandlung des Patienten bestimmt wird.

10. Verfahren gemäß einem der Ansprüche 1 bis 9, das weiterhin Folgendes umfasst:
Bestimmen der Konzentration eines weiteren Neurotrophins in einer Probe, die aus Liquor des Patienten erhalten wurde; und
Vergleichen der Konzentration mit einem Referenzwert, der einen bekannten Krankheits- oder Gesundheitszustand darstellt;
wobei eine veränderte Konzentration des weiteren Neurotrophins in dem Liquor von dem Patienten im Vergleich zu dem Referenzwert, der einen bekannten Gesundheitszustand darstellt, eine Diagnose oder Prognose oder ein erhöhtes Risiko für Alzheimer-Krankheit bei dem Patienten anzeigt.

11. Verfahren gemäß Anspruch 10, wobei es sich bei dem Neurotrophin um Neurotrophin-3 handelt.

12. Verfahren gemäß Anspruch 11, wobei eine Konzentration von Neurotrophin-3 von ≥ 15 pg/ml in dem Liquor eine Diagnose oder Prognose oder ein erhöhtes Risiko für Alzheimer-Krankheit bei dem Patienten anzeigt.

## Revendications

1. Procédé pour diagnostiquer ou pronostiquer la maladie d'Alzheimer chez un sujet, ou pour déterminer si un sujet présente un risque accru de développer la maladie d'Alzheimer, comprenant :
- la détermination d'un taux en facteur de croissance des nerfs dans un échantillon prélevé dans le liquide céphalorachidien dudit sujet ;
- et la comparaison dudit taux à une valeur de référence représentant une maladie ou un état de santé connu,
dans lequel le taux en facteur de croissance des nerfs ≥ 4 pg/mL dans ledit liquide céphalorachidien prélevé sur ledit sujet indique un diagnostic, ou un pronostic, ou un risque accru de ladite maladie d'Alzheimer chez ledit sujet.

2. Procédé pour contrôler la progression de la maladie d'Alzheimer chez un sujet, comprenant :
- la détermination d'un taux en facteur de croissance des nerfs dans un échantillon prélevé dans le liquide céphalorachidien dudit sujet ;
- et la comparaison dudit taux à une valeur de référence représentant une maladie ou un état de santé connu,
dans lequel un taux en facteur de croissance des nerfs ≥ 4 pg/mL dans ledit liquide céphalorachidien prélevé sur ledit sujet indique un diagnostic, ou un pronostic, ou un risque accru de ladite maladie d'Alzheimer chez ledit sujet.

3. Utilisation du procédé selon la revendication 1 ou 2 pour évaluer un traitement de la maladie d'Alzheimer.

4. Procédé selon la revendication 1 à 3, dans lequel un taux en facteur de croissance des nerfs dans l'intervalle de 4 pg/mL à 25 pg/mL, en particulier dans la gamme de 4 pg/mL à 14 pg/mL, dans ledit liquide céphalorachidien indique un diagnostic, ou un pronostic, ou un risque accru de maladie d'Alzheimer chez ledit sujet.

5. Procédé selon l'une quelconque des revendications 1 à 4, dans lequel ledit sujet est un être humain.

6. Procédé selon l'une quelconque des revendications 1 à 5, dans lequel le facteur de croissance des nerfs est détecté au moyen d'un dosage immunologique, d'un dosage biologique et/ou d'un dosage par liaison.

7. Procédé selon l'une quelconque des revendications 1 à 6, comprenant en outre la comparaison d'un taux en facteur de croissance des nerfs dans ledit échantillon avec un taux dans une série d'échantillons prélevés sur ledit sujet au cours d'une certaine période.

8. Procédé selon l'une quelconque des revendications 1 à 7, dans lequel ledit sujet reçoit un traitement avant un ou plusieurs prélèvements d'échantillon.

9. Procédé selon l'une quelconque des revendications 1 à 8, dans lequel ledit taux dans lesdits échantillons est déterminé avant et après ledit traitement dudit sujet.

10. Procédé selon l'une quelconque des revendications 1 à 9, comprenant en outre :
- la détermination d'un taux en une autre neurotrophine dans un échantillon prélevé dans le liquide céphalorachidien dudit sujet ;
- et la comparaison dudit taux à une valeur de référence représentant une maladie ou un état de santé connu ;
dans lequel un taux en ladite autre neurotrophine dans ledit liquide céphalorachidien prélevé sur ledit sujet différent de ladite valeur de référence représentant un état de santé connu indique un diagnostic, ou un pronostic, ou un risque accru de maladie d'Alzheimer chez ledit sujet.

11. Procédé selon la revendication 10, dans lequel ladite neurotrophine est la neurotrophine 3.

12. Procédé selon la revendication 11, dans lequel un taux en neurotrophine 3 ≥ 15 pg/mL dans ledit liquide céphalorachidien indique un diagnostic, ou un pronostic, ou un risque accru de maladie d'Alzheimer chez ledit sujet.
